# EUROPÄISCHE PATENTANMELDUNG

(11) **EP 1 234 613 A1**
(43) Veröffentlichungstag der Anmeldung: **28.08.2002**
(21) Anmeldenummer: 02002072.3
(22) Anmeldetag: 11.02.2002
(51) Int. Cl.: B01J 31/18, C07C 17/358

(54) **Katalysatorgemisch zur Isomerisierung von Dichlorbutenen**

(30) Priorität: 22.02.2001 DE 10108619
(71) Anmelder: BAYER AG, 51368 Leverkusen (DE)
(72) Erfinder: Schertl, Peter, Dr., 45772 Marl (DE)

(57) **Zusammenfassung**

Die Erfindung betrifft eine Zusammensetzung herstellbar durch Mischung eines aromatischen Amins oder mehrerer aromatischer Amine der allgemeinen Formel I, 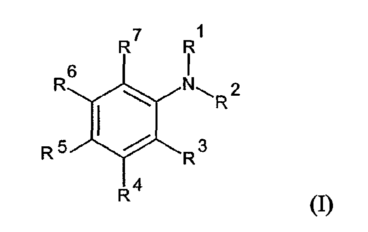 wobei
- R¹ bis R⁷: unabhängig voneinander für H, C₁- bis C₁₂-Alkyl, C₅- bis C₈-Cycloalkyl, C₆- bis C₁₄-Aryl, Alkylaryl, Arylalkyl, wobei zwei benachbarte Reste gemeinsam gesättigte oder ungesättigte C₃- bis C₁₄-Cyclen bilden können, oder für -SiR⁸R⁹R¹⁰, wobei R⁸ bis R¹⁰ unabhängig voneinander C₁- bis C₄-Alkyl, C₅- bis C₈-Cycloalkyl oder C₆- bis C₁₄-Aryl bedeuten kann,
mit einem wasserfreien Kupfersalz CuXₙ, wobei n = 1 oder 2 und X = Halogenid, Sulfat, Acetat, Acetylacetonat ist, oder einem Gemisch aus zwei oder mehreren dieser Verbindungen in 1,4-Dichlor-2-buten oder 3,4-Dichlor-1-buten,
einem Verfahren zu deren Herstellung, deren Verwendung als Katalysator, sowie einem Verfahren zur Isomerisierung von 1,4-Dichlor-2-buten zu 3,4-Dichlor-1-buten oder umgekehrt unter Verwendung der erfindungsgemäßen Zusammensetzung.

## Beschreibung

Die Erfindung betrifft eine Zusammensetzung herstellbar durch Mischung eines aromatischen Amins oder mehrerer aromatischer Amine der allgemeinen Formel I, 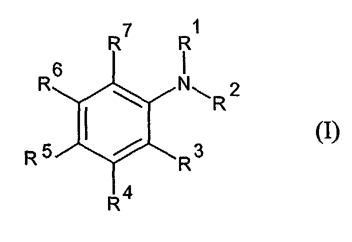 wobei
- R¹ bis R⁷: unabhängig voneinander für H, C₁- bis C₁₂-Alkyl, C₅- bis C₈-Cycloalkyl, C₆- bis C₁₄-Aryl, Alkylaryl, Arylalkyl, wobei zwei benachbarte Reste gemeinsam gesättigte oder ungesättigte C₃- bis C₁₄-Cyclen bilden können, oder für -SiR⁸R⁹R¹⁰, wobei R⁸ bis R¹⁰ unabhängig voneinander C₁- bis C₄-Alkyl, C₅- bis C₈-Cycloalkyl oder C₆- bis C₁₄-Aryl bedeuten kann,
mit einem wasserfreien Kupfersalz CuXₙ, wobei n = 1 oder 2 und X = Halogenid, Sulfat, Acetat, Acetylacetonat, Naphthenat ist, oder einem Gemisch aus zwei oder mehreren dieser Kupfersalze in 1,4-Dichlor-2-buten oder 3,4-Dichlor-1-buten, einem Verfahren zu deren Herstellung, deren Verwendung als Katalysator, sowie einem Verfahren zur Isomerisierung von 1,4-Dichlor-2-buten zu 3,4-Dichlor-1-buten oder umgekehrt unter Verwendung der erfindungsgemäßen Zusammensetzung.

3,4-Dichlor-1-buten ist ein wichtiges Zwischenprodukt bei der Herstellung von 2-Chloropren, das als Monomer für die Herstellung von Polychloroprenkautschuk großtechnisch Verwendung findet.

Bei der Chlorierung von Butadien entsteht ein Gemisch von *cis*-1,4-Dichlor-2-buten, *trans*-1,4-Dichlor-2-buten und 3,4-Dichlor-1-buten, das etwa 65 % *cis-* bzw. *trans*-1,4-Dichlor-2-buten und etwa 35 % 3,4-Dichlor-1-buten enthält. Diese Isomeren liegen gewöhnlich in der Mischung im Gleichgewicht vor, wobei das Verhältnis von den Herstellungsbedingungen abhängt. Im folgenden seien zur Vereinfachung *cis*und *trans*-1,4-Dichlor-2-buten als 1,4-Dichlor-2-buten zusammengefasst. Aufgrund der unterschiedlichen Siedepunkte (1,4-Dichlor-2-buten: 154-9°C und 3,4-Dichlor-1-buten: 123°C) kann dieses Gemisch destillativ aufgetrennt werden. Da für die Herstellung von 2-Chloropren nur das 3,4-Dichlor-1-buten geeignet ist, muss das 1,4-Dichlor-2-buten zum 3,4-Dichlor-1-buten isomerisiert und in den Prozess zurückgeführt werden.

Die üblichen Verfahren zur Isomerisation von 1,4-Dichlor-2-buten zu 3,4-Dichlor-1-buten oder umgekehrt basieren auf der Verwendung geeigneter Isomerisierungskatalysatoren, welche für eine schnelle Gleichgewichtseinstellung zwischen den Isomeren in 1,4-Dichlor-2-buten oder 3,4-Dichlor-1-buten bei erhöhten Temperaturen sorgen. In den meisten Verfahren werden Metallsalze des Kupfers in Gegenwart basischer Zusätze eingesetzt, die zur Erhöhung der Reaktionsgeschwindigkeiten dienen.

DE-A-21 38 790 offenbart ein Verfahren zur Isomerisierung von 1,4-Dichlor-2-buten zu 3,4-Dichlor-1-buten oder umgekehrt bei 80 bis 160°C mittels Kupfernaphthenat, Dinitril und Amid. In DE-A-21 43 157 wird ein Verfahren zur Isomerisierung in Gegenwart von Kupfersalzen und Oxim-Derivaten bei 80 bis 160°C beschrieben. DE-A-22 00 780 beansprucht ein Verfahren, das als Katalysator ein Gemisch aus einer Kupferverbindung und einer organischen Phosphorverbindung enthält. DE-A-21 07 468 offenbart die Verwendung von Kupfernaphthenat und Nitroverbindungen, DE-A-21 30 488 die Verwendung von Kupfernaphthenat und Nitroanilinen. In DE-A-22 12 235 wird ein Isomerisierungsverfahren mittels Kupferverbindung und Harnstoffderivat beschrieben. DE-A-22 06 971 beansprucht die Verwendung eines Gemisches aus Kupferverbindung (Kupfer-II-Stearat, -oleat und -naphthenat) und chlorhaltigem Anilinderivat. In US-A-4,895,993 wird ein Verfahren zur Isomerisierung in Gegenwart eines Katalysators bestehend aus einer Kupferverbindung und einem Dithiocarbamat- oder Trithiocarbonat- Derivat.

Rostovshchikova et al. beschreiben in *Zh. Obshch. Khim.* **1994,** *64,* 12 die Verwendung von Triphenylphosphin oder in *Kinet. Katal.* **1992,** *33,* 314 den Einsatz unterschiedlicher Dialkylsulfide in Gegenwart von Kupferhalogeniden zur katalytischen Isomerisierung. Asatryan et al. untersuchten in *Arm. Khim. Zh.* **1988,** *41,* 278 die Wirkung von makrozyklischen Polyethern oder Polyethylenglykolen, in *Arm. Khim. Zh.* **1988,** *41,* 273 den Einfluss von Benzonitril, Nitrobenzol, DMF, Dimethylsulfon oder Acetophenon.

Von Nachteil an diesen Verfahren ist, dass die Umwandlungsgeschwindigkeiten vergleichsweise gering sind und eine große Menge an unerwünschten Nebenprodukten gebildet wird.

In *Arm. Khim. Zh.* **1987,** *40,* 709 wird von Asatryan et al. die Wirkung unterschiedlicher Isomerisierungskatalysatoren auf der Basis von Halogenidsalzen des Kupfers, Eisens oder Zinks in Gegenwart von Amin-Derivaten wie Triethylamin, Diethylamin, Triethanolamin, Ethylendiamin oder Anilin beschrieben. Asatryan et al. beschreiben jedoch, dass aliphatische Amine zu Isomerisierungskatalysatoren führen, die aufgrund ihrer höherer Nucleophilie eine höhere Effektivität zeigen als aromatische Amine. Daher wird dort die Verwendung aliphatischer Amine empfohlen.

Es war nun Aufgabe der vorliegenden Erfindung ein wirtschaftliches Verfahren zur Isomerisierung von 1,4-Dichlor-2-buten zu 3,4-Dichlor-1-buten oder umgekehrt mittels eines Katalysatorsystems zu finden, welches hohe Umwandlungsgeschwindigkeiten gewährleistet und mit reduzierter Nebenproduktbildung die Isomerisierung katalysiert.

Überraschend wurde gefunden, dass bei einem Verfahren zur Isomerisierung von 1,4-Dichlor-2-buten zu 3,4-Dichlor-1-buten oder umgekehrt unter Verwendung einer Zusammensetzung herstellbar durch Mischung eines aromatischen Amins und eines Kupfersalzes die Isomerisierungsgeschwindigkeit entgegen den Befunden von Asatryan et al. in *Arm. Khim. Zh.* **1987,** *40,* 709 höher, die Nebenproduktbildung aufgrund der geringeren Basizität der aromatischen Amine geringer und damit das Verfahren wirtschaftlicher ist.

Gegenstand der Erfindung ist eine Zusammensetzung herstellbar durch Mischung eines aromatischen Amins oder mehrerer aromatischer Amine der allgemeinen Formel I, 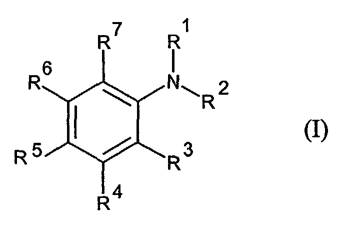 wobei
- R¹ bis R⁷: unabhängig voneinander für H, C₁- bis C₁₂-Alkyl, C₅- bis C₈-Cycloalkyl, C₆- bis C₁₄-Aryl, Alkylaryl, Arylalkyl steht, wobei zwei benachbarte Reste gemeinsam gesättigte oder ungesättigte C₃- bis C₁₄-Cyclen bilden können, oder für -SiR⁸R⁹R¹⁰, wobei R⁸ bis R¹⁰ unabhängig voneinander C₁- bis C₄-Alkyl, C₅- bis C₈-Cycloalkyl oder C₆- bis C₁₄-Aryl bedeuten kann,
mit einem wasserfreien Kupfersalz CuXₙ, wobei n = 1 oder 2 und X = Halogenid, Sulfat, Acetat, Acetylacetonat, oder Naphthenat ist, oder einem Gemisch aus zwei oder mehreren dieser Kupfersalze.

Unter C₁-C₁₂-Alkyl werden sämtliche dem Fachmann bekannte lineare oder verzweigte, gesättigte oder ungesättigte Alkylreste mit 1 bis 12 C-Atomen verstanden, wie Methyl, Ethyl, n-Propyl, i-Propyl, n-Butyl, i-Butyl, t-Butyl, n-Pentyl, i-Pentyl, neo-Pentyl, n-Hexyl, i-Hexyl, Heptyl, Octyl, Nonyl, Decyl, Undecyl und Dodecyl, sowie deren ungesättigten Homologen.

Unter C₅- bis C₈-Cycloalkyl werden sämtliche dem Fachmann bekannte cyclische Alkylreste mit 5 bis 8 C-Atomen verstanden, wie Cyclopentyl, Cyclohexyl, Cycloheptyl, Cyclooctyl, sowie deren ungesättigten Homologen.

Unter C₆- bis C₁₄-Aryl werden sämtliche dem Fachmann bekannte Arylreste mit 6 bis 14 C-Atomen verstanden, wie Phenyl, Naphthenyl, Fluorenyl, Anthracenyl und Phenanthranyl.

Bevorzugte aromatische Amine sind Anilin, N,N-Dimethylanilin, p-Toluidin oder Gemische aus 2 oder 3 dieser Komponenten.

Das molare Verhältnis der beiden Komponenten CuXₙ, wobei n = 1 oder 2 und X = Halogenid, Sulfat, Acetat, Acetylacetonat, Naphthenat ist, zu aromatischem Amin liegt dabei vorteilhaft im Bereich von 1:0,5 bis 1,5, bevorzugt 1:0,7 bis 1:1 Die Konzentration der beiden Komponenten liegt vorteilhaft im Bereich von 10⁻³ bis 1 mol/l, bevorzugt zwischen 5×10⁻² bis 5×10⁻¹ mol Cu/l.

Die Erfindung betrifft weiterhin ein Verfahren zur Herstellung einer erfindungsgemäßen Zusammensetzung, dadurch gekennzeichnet, dass man die Komponenten in der Reihenfolge aromatisches Amin oder aromatische Amine und anschließend das oder die wasserfreien Kupfersalze mischt. Es ist vorteilhaft, diesen Mischprozess unter Schutzgasatmosphäre, wie Stickstoffatmosphäre oder Argonatmosphäre, im Temperaturbereich von 30 bis 180°C, bevorzugt 50 bis 150°C durchzuführen.

Die Erfindung betrifft weiterhin die Verwendung der erfindungsgemäßen Zusammensetzung als Katalysator, insbesondere als Katalysator in einem Verfahren zur Isomerisierung von 1,4-Dichlor-2-buten zu 3,4-Dichlor-1-buten oder von 3,4-Dichlor-1-buten zu 1,4-Dichlor-2-buten.

Somit ist ein weiterer Gegenstand der Erfindung ein Verfahren zur Isomerisierung von 1,4-Dichlor-2-buten zu 3,4-Dichlor-1-buten oder von 3,4-Dichlor-1-buten zu 1,4-Dichlor-2-buten, dadurch gekennzeichnet, dass man
a) bei einer Temperatur von 30 bis 180°C, bevorzugt im Bereich von 50° bis 150°C, zu 1,4-Dichlor-2-buten, 3,4-Dichlor-1-buten oder einem Gemisch hieraus ein aromatisches Amin oder mehrere aromatische Amine der allgemeinen Formel I, 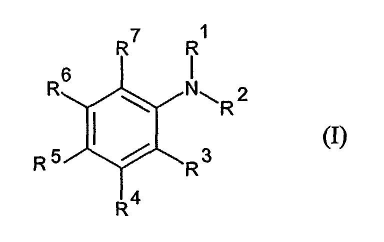 wobei
   - R¹ bis R⁷: unabhängig voneinander für H, C₁- bis C₁₂-Alkyl, C₅- bis C₈-Cyclo-alkyl, C₆- bis C₁₄-Aryl, Alkylaryl, Arylalkyl steht, wobei zwei benachbarte Reste gemeinsam gesättigte oder ungesättigte C₃- bis C₁₄-Cyclen bilden können, oder für -SiR⁸R⁹R¹⁰, wobei R⁸ bis R¹⁰ unabhängig voneinander C₁- bis C₄-Alkyl, C₅- bis C₈-Cycloalkyl oder C₆- bis C₁₄-Aryl bedeuten kann,
   und ein wasserfreies Kupfersalz CuXₙ, wobei n = 1 oder 2 und X = Halogenid, Sulfat, Acetat, Acetylacetonat, Naphthenat ist, oder ein Gemisch aus zwei oder mehreren dieser Verbindungen zusetzt.
b) die Reaktionslösung solange reagieren lässt, bis sich ein Gleichgewicht zwischen 1,4-Dichlor-2-buten und 3,4-dichlor-1-buten eingestellt hat, bevorzugt zwischen 1 und 180 Minuten, besonders bevorzugt zwischen 15 und 45 Minuten,
c) man ein Gemisch aus 1,4-Dichlor-2-buten und 3,4-Dichlor-1-buten kontinuierlich entfernt und dieses anschließend destillativ trennt,
d) dem Reaktionssystem die nicht gewünschte Komponente aus der in c) durchgeführten Destillation erneut zuführt
e) und gegebenenfalls dem Reaktionssystem gleichzeitig mit c) kontinuierlich 1,4-Dichlor-2-buten und/oder 3,4-Dichlor-1-buten zuleitet.

Das Verfahren kann sowohl diskontinuierlich als auch kontinuierlich zwischen 0,01 bar und 10 bar, bevorzugt zwischen 0,1 und 1,0 bar, erfolgen, wobei es sich empfiehlt, zunächst eine höherkonzentrierte Lösung des Katalysatorsystems, bevorzugt 10⁻¹ bis 1 mol Cu/l, in 1,4-Dichlor-2-buten und/oder 3,4-Dichlor-1-buten herzustellen und diese kontinuierlich einer größeren Menge an 1,4-Dichlor-2-buten und/oder 3,4-Dichlor-1-buten zuzugeben, so dass die gewünschte Konzentration bevorzugt 5 x 10⁻² bis 5 x 10⁻¹ mol Cu/l des Katalysators erhalten wird, wobei kontinuierlich 1,4-Dichlor-2-buten und/oder 3,4-Dichlor-1-buten zugeleitet, ein Gemisch an 1,4-Dichlor-2-buten und 3,4-Dichlor-1-buten kontinuierlich entfernt und anschließend destillativ getrennt wird.

Im folgenden wird die Erfindung anhand von Ausführungsbeispielen näher erläutert, ohne diese jedoch auf die Beispiele einzuschränken.

### Beispiele

### Herstellung der Katalysatormischungen

### Katalysatormischung A

Unter Stickstoffatmosphäre werden in einem 500 ml Rundkolben mit Innenthermometer, Rückflusskühler und Überdruckventil 232.0 g 1,4-Dichlor-2-buten vorgelegt, bei 50°C 9,67 g (104 mmol) Anilin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung B

In der bei der Herstellung von Katalysator A beschriebenen Apparatur werden unter Stickstoff 232.0 g 1,4-Dichlor-2-buten vorgelegt, bei 50°C 8,72 g (94 mmol) Anilin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung C

In der bei der Herstellung von Katalysator A beschriebenen Apparatur werden unter Stickstoff 232.0 g 1,4-Dichlor-2-buten vorgelegt, bei 50°C 7,75 g (83 mmol) Anilin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysator D

In der bei der Herstellung von Katalysator A beschriebenen Apparatur werden unter Stickstoff 232.0 g 1,4-Dichlor-2-buten vorgelegt, bei 50°C 6,78 g (73 mmol) Anilin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung E

In der bei der Herstellung von Katalysator A beschriebenen Apparatur werden unter Stickstoff 232.0 g 1,4-Dichlor-2-buten vorgelegt, bei 50°C 11,34 g (94 mmol) N,N-Dimethylanilin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung F

In der bei der Herstellung von Katalysator A beschriebenen Apparatur werden unter Stickstoff 232.0 g 1,4-Dichlor-2-buten vorgelegt, bei 50°C 15,84 g (94 mmol) Diphenylamin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung G

In der bei der Herstellung von Katalysator A beschriebenen Apparatur werden unter Stickstoff 232.0 g 1,4-Dichlor-2-buten vorgelegt, bei 50°C 10,52 g (104 mmol) Triethylamin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung H

In der bei der Herstellung von Katalysator A beschriebenen Apparatur werden unter Stickstoff 232.0 g 1,4-Dichlor-2-buten vorgelegt, bei 50°C 9,47 g (94 mmol) Triethylamin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Katalysatormischung I

In der bei der Herstellung von Katalysator A beschriebenen Apparatur werden unter Stickstoff 232.0 g 1,4-Dichlor-2-buten vorgelegt, bei 50°C 7,37 g (73 mmol) Triethylamin zugegeben und 4 h bei dieser Temperatur gerührt. Anschließend werden bei 50°C 10,29 g (104 mmol) Kupfer(I)-chlorid zugegeben und weitere 8 h bei dieser Temperatur gerührt.

### Durchführung der Isomerisierungsreaktionen

### Beispiel 1

In einem 500 ml Rundkolben mit Innenthermometer, Rückflusskühler und Überdruckventil werden unter Stickstoff 240,0 g 1,4-Dichlor-2-buten vorgelegt, bei 130°C 84,0 g der auf Raumtemperatur erhaltenen Katalysatormischung A zugegeben, so dass sich unter starkem Rühren eine Mischungstemperatur von 105°C einstellt, und bei dieser Temperatur 90 min gerührt.

Während der Reaktionsdauer werden in festgelegten Intervallen Proben entnommen und gaschromatographisch auf ihren Gehalt an 3,4-Dichlor-1-buten, *cis*-1,4-Dichlor-2-buten, *trans*-1,4-Dichlor-2-buten und evtl. gebildeten Nebenprodukten, wie 1-Chloropren, hin untersucht. Der Startpunkt der Reaktion wird durch die beendete Zugabe der Katalysatormischung festgelegt. Es werden zum Startpunkt, nach 5, 15, 30 und 90 Minuten Proben von jeweils ca. 2 ml Volumen entnommen.

### Beispiel 2

In dem in Beispiel 1 beschriebenen Versuchsaufbau werden 240,0 g 1,4-Dichlor-2-buten vorgelegt, bei 130°C 84,0 g der Katalysatormischung B zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 3

In dem in Beispiel 1 beschriebenen Versuchsaufbau werden 240,0 g 1,4-Dichlor-2-buten vorgelegt, bei 130°C 84,0 g der Katalysatormischung C zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 4

In dem in Beispiel 1 beschriebenen Versuchsaufbau werden 240,0 g 1,4-Dichlor-2-buten vorgelegt, bei 130°C 84,0 g der Katalysatormischung D zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 5

In dem in Beispiel 1 beschriebenen Versuchsaufbau werden 240,0 g 1,4-Dichlor-2-buten vorgelegt, bei 130°C 84,0 g der Katalysatormischung E zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 6

In dem in Beispiel 1 beschriebenen Versuchsaufbau werden 240,0 g 1,4-Dichlor-2-buten vorgelegt, bei 130°C 84,0 g der Katalysatormischung F zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 7

In dem in Beispiel 1 beschriebenen Versuchsaufbau werden 240,0 g 1,4-Dichlor-2-buten vorgelegt, bei 130°C 84,0 g der Katalysatormischung G zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 8

In dem in Beispiel 1 beschriebenen Versuchsaufbau werden 240,0 g 1,4-Dichlor-2-buten vorgelegt, bei 130°C 84,0 g der Katalysatormischung H zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 9

In dem in Beispiel 1 beschriebenen Versuchsaufbau werden 240,0 g 1,4-Dichlor-2-buten vorgelegt, bei 130°C 84,0 g der Katalysatormischung I zugegeben, so dass sich eine Mischungstemperatur von 105°C einstellt, und bei dieser Temperatur 90 min gerührt. Zeitpunkt, Probennahme und Untersuchung der Proben erfolgen wie in Beispiel 1 erläutert.

### Beispiel 10

In dem in Beispiel 1 beschriebenen Versuchsaufbau werden 240,0 g 1,4-Dichlor-2-buten vorgelegt, bei 165°C 84,0 g der Katalysatormischung H zugegeben, so dass sich eine Mischungstemperatur von 130°C einstellt, und bei dieser Temperatur 90 min gerührt. Zeitpunkt, Probennahme und Untersuchung der Proben erfolgen wie in Beispiel 1 erläutert.

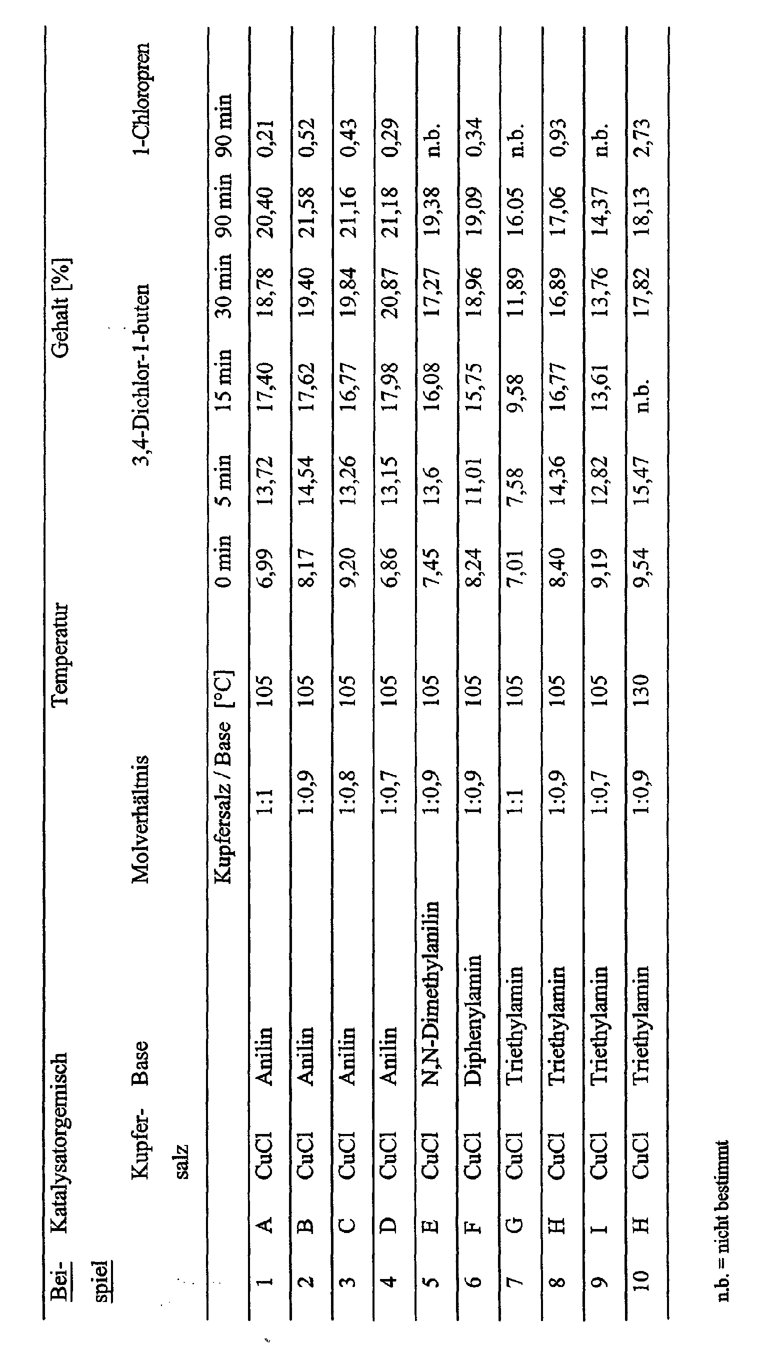

## Patentansprüche

1. Zusammensetzung herstellbar durch Mischung eines aromatischen Amins oder mehrerer aromatischer Amine der allgemeinen Formel I, 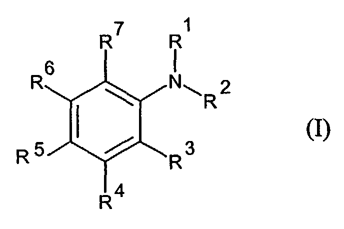 wobei
R¹ bis R⁷ unabhängig voneinander für H, C₁- bis C₁₂-Alkyl, C₅- bis C₈-Cyclo-alkyl, C₆- bis C₁₄-Aryl, Alkylaryl, Arylalkyl steht, wobei zwei benachbarte Reste gemeinsam gesättigte oder ungesättigte C₃- bis C₁₄-Cyclen bilden können, oder für -SiR⁸R⁹R¹⁰, wobei R⁸ bis R¹⁰ unabhängig voneinander C₁- bis C₄-Alkyl, C₅- bis C₈-Cycloalkyl oder C₆- bis C₁₄-Aryl bedeuten kann,
mit einem wasserfreien Kupfersalz CuXₙ, wobei n = 1 oder 2 und X = Halogenid, Sulfat, Acetat, Acetylacetonat, Naphthenat ist, oder einem Gemisch aus zwei oder mehreren dieser Kupfersalze.

2. Zusammensetzung gemäss Anspruch 1, **dadurch gekennzeichnet, dass** man als Amin Anilin, N,N-Dimethylanilin, p-Toluidin oder Gemische aus 2 oder 3 dieser Komponenten einsetzt.

3. Zusammensetzung nach Anspruch 1 oder 2, **dadurch gekennzeichnet, dass** das molare Verhältnis der beiden Komponenten Kupfersalz/Kupfersalzen zu aromatischem Amin/Aminen im Bereich von 1:0,5 bis 1,5 liegt.

4. Zusammensetzung nach einem der Ansprüche 1 bis 3, **dadurch gekennzeichnet, dass** die Konzentration der Komponenten Kupfersalz/Kupfersalzen und aromatischem Amin/Aminen im Bereich von 10⁻³ bis 1 mol/l beträgt.

5. Verwendung der Zusammensetzung nach einem der Ansprüche 1 bis 4 als Katalysator.

6. Verfahren zur Isomerisierung von 1,4-Dichlor-2-buten zu 3,4-Dichlor-1-buten oder von 3,4-Dichlor-1-buten zu 1,4-Dichlor-2-buten, **dadurch gekennzeichnet, dass** man
a) bei einer Temperatur von 30 bis 180°C zu 1,4-Dichlor-2-buten, 3,4-Dichlor-1-buten oder einem Gemisch hieraus ein aromatisches Amin oder mehrere aromatische Amine der allgemeinen Formel I, 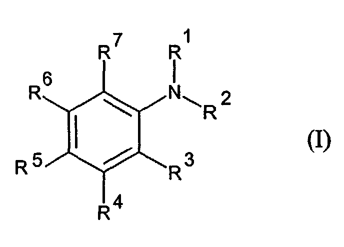 wobei
R¹ bis R⁷ unabhängig voneinander für H, C₁- bis C₁₂-Alkyl, C₅- bis C₈-Cycloalkyl, C₆- bis C₁₄-Aryl, Alkylaryl, Arylalkyl steht, wobei zwei benachbarte Reste gemeinsam gesättigte oder ungesättigte C₃- bis C₁₄-Cyclen bilden können, oder für -SiR⁸R⁹R¹⁰, wobei R⁸ bis R¹⁰ unabhängig voneinander C₁- bis C₄-Alkyl, C₅- bis C₈-Cycloalkyl oder C₆- bis C₁₄-Aryl bedeuten kann,
und ein wasserfreies Kupfersalz CuXₙ, wobei n = 1 oder 2 und X = Halogenid, Sulfat, Acetat, Acetylacetonat ist, oder ein Gemisch aus zwei oder mehreren dieser Kupfersalze zusetzt,
b) die Reaktionslösung solange reagieren lässt, bis sich ein Gleichgewicht zwischen 1,4-Dichlor-2-buten und 3,4-dichlor-1-buten eingestellt hat,
c) man ein Gemisch aus 1,4-Dichlor-2-buten und 3,4-Dichlor-1-buten kontinuierlich entfernt und dieses anschließend destillativ trennt,
d) dem Reaktionssystem die nicht gewünschte Komponente aus der in c) durchgeführten Destillation erneut zuführt
e) und gegebenenfalls dem Reaktionssystem gleichzeitig mit c) kontinuierlich 1,4-Dichlor-2-buten und/oder 3,4-Dichlor-1-buten zuleitet.

7. Verfahren gemäss Anspruch 6, **dadurch gekennzeichnet, dass** die Temperatur im Schritt a) im Bereich von 50 bis 150°C liegt.

8. Verfahren gemäss Anspruch 6 oder 7, **dadurch gekennzeichnet, dass** die Reaktionszeit im Schritt b) im Bereich von 15 und 45 Minuten beträgt.

9. Verfahren gemäss einem der Ansprüche 6 bis 8, **dadurch gekennzeichnet, dass** man im Schritt a) eine vorbereitete Zusammensetzung gemäss einem der Ansprüche 1 bis 5 in Konzentrationen im Bereich von 10⁻¹ bis 1 mol Cu/l in 1,4-Dichlor-2-buten und/oder 3,4-Dichlor-1-buten einsetzt und diese zu 1,4-Dichlor-2-buten, 3,4-Dichlor-1-buten oder einem Gemisch hieraus zugibt.

10. Verfahren zur Herstellung einer Zusammensetzung gemäss einem der Ansprüche 1 bis 5, **dadurch gekennzeichnet, dass** man die einzelnen Komponenten miteinander vermischt.
